# EUROPÄISCHE PATENTSCHRIFT

(11) **EP 3 668 671 B1**
(45) Veröffentlichungstag und Bekanntmachung des Hinweises auf die Patenterteilung: **03.11.2021**
(21) Anmeldenummer: 18749299.6
(22) Anmeldetag: 18.07.2018
(51) Int. Cl.: B23B 45/00, G01N 33/46

(54) **BOHRVORRICHTUNG UND VERFAHREN ZUR DURCHFÜHRUNG VON BOHRUNGEN AN SCHWER ZUGÄNGLICHEN STELLEN**
DRILLING DEVICE AND METHOD FOR CARRYING OUT DRILLING AT LOCATIONS WHICH ARE DIFFICULT TO REACH
DISPOSITIF DE PERÇAGE ET PROCÉDÉ POUR L'EXÉCUTION DE PERÇAGES DANS DES EMPLACEMENTS DIFFICILES D'ACCÈS

(30) Priorität: 17.08.2017 DE 102017007725
(43) Veröffentlichungstag der Anmeldung: 24.06.2020
(73) Patentinhaber: IML Instrumenta Mechanik Labor GmbH, 69168 Wiesloch (DE)
(72) Erfinder: HUNGER, Erich, 76133 Karlsruhe (DE); HUNGER, Sebastian, 69181 Leimen (DE); HUNGER, Fabian, 69181 Leimen (DE)
(74) Vertreter: mepat Patentanwälte
(86) Internationale Anmeldenummer: PCT/EP2018/000360
(87) Internationale Veröffentlichungsnummer: WO 2019/034272

(56) Entgegenhaltungen:
- DE-A1- 10 031 395
- US-A- 4 541 423
- US-A1- 2011 168 419

## Beschreibung

Die Erfindung betrifft eine Bohrvorrichtung, die einen Umlenkaufsatz aufweist. Ferner betrifft die Erfindung ein Verfahren zur Durchführung von Bohrungen oder Bohrwiderstandsmessungen an schwer zugänglichen Stellen.

Aus dem Stand der Technik ist bekannt, die Beschaffenheit von Körpern mit säulenförmigen oder zylindrischen Querschnitten, insbesondere von Bäumen und Holzmasten, zur Detektion möglicher Defekte wie Fäulnis oder ähnlichem mittels Bohrwiderstandsmessung zu untersuchen. Hierbei kann nach einem Verfahren des Standes der Technik die Bohrnadel eines Bohrwiderstandsmessgeräts im Wesentlichen senkrecht zur Stamm-/ Mastachse in definierter Weise eindringen gelassen und der dabei von dem Holz ausgeübte Widerstand gemessen werden. Starke Abweichungen von einem erwartungsgemäßen Widerstand oder ein abrupter Abfall des mechanischen Widerstands während des Einführens deuten auf einen beispielsweise durch Aushöhlung oder Fäulnis bedingten Defekt. Eine Vorrichtung zur Durchführung von solchen Bohrwiderstandsmessungen ist aus der DE 10 031 395 A1 bekannt, die eine Bohrvorrichtung nach dem Oberbegriff des Anspruchs 1 offenbart.

Ein Aufsatzgerät für elektrische Handbohrmaschinen, das zur Durchführung von Untersuchungen wie beispielsweise Dichtemessungen an Bäumen dient, ist unter anderem aus der DE 297 07 307 U1 bekannt. Dort wird ein Bohraufsatz mit einer handelsüblichen Bohrmaschine direkt oder über einen Adapter verbunden und über eine Profilwelle gekoppelt, sodass die Energie zum Antrieb des Bohraufsatzes auf dessen Antriebsmechanismus übertragen werden kann.

Aus der DE 10 2016 011 776 B3 ist ein Gerät zur Durchführung solcher Bohrwiderstandsmessungen sowie eine Nadelführungsvorrichtung dafür bekannt Dabei wird die Nadelführungsvorrichtung mit dem Antrieb des Bohrwiderstandsmessgerätes gekoppelt und ist dazu ausgebildet, die Nadel mittels eines Scherengitters während des Bohrvorgangs zu stützen und durch fluchtend angeordnete Durchtrittsöffnungen zu führen.

Zur Durchführung von Bohrwiderstandsmessungen an einer schwer zugänglichen Stelle wie im Bereich der Erdgleiche offenbart DE 10 2013 001 711 A1 Bohrwiderstandsmessungen unter einem vorgegebenen, von 90° in Bezug auf die Stamm-/Mastachse abweichenden Einführungswinkel durchzuführen. Die dabei erhaltenen Ergebnisse sind allerdings schlechter vergleichbar und erfordern eine komplexere Auswertesoftware.

Abgesehen von Bohrwiderstandsmessungen sind auch Fälle denkbar, bei denen keine Widerstandsmessung erfolgen soll, sondern lediglich Bohrungen in ein Objekt an einer unzugänglichen Stelle eingebracht werden sollen, etwa um zur Vorbeugung oder Behandlung von Defekten wie Fäulnis in Holz eine Schutz-, Imprägnier- oder Behandlungsflüssigkeit zu injizieren.

Ausgehend von diesem Stand der Technik ist es Aufgabe der vorliegenden Erfindung, eine Vorrichtung zur Durchführung von Bohrungen oder Bohrwiderstandsmessungen auch an schwer zugänglichen Stellen bereitzustellen.

Diese Aufgabe wird durch eine Bohrvorrichtung mit den Merkmalen des Anspruchs 1 gelöst.

Das mit den Merkmalen des Anspruchs 7 offenbarte Verfahren löst die Aufgabe, die Durchführung von Bohrungen oder Bohrwiderstandsmessungen an schwer zugänglichen - Stellen zu ermöglichen.

Weiterbildungen und bevorzugte Ausführungsformen der Bohrvorrichtung und des Verfahrens sind in den jeweiligen Unteransprüchen ausgeführt.

Ein Gegenstand der Erfindung ist eine Bohrvorrichtung zur Durchführung von Bohrungen an schwer zugänglichen Stellen, wobei die Bohrvorrichtung in einer ersten Ausführungsform ein Bohrgerät, eine in dem Bohrgerät gelagerte und geführte Bohrnadel, die in der Anwendung zum frontseitigen Austritt aus dem Gehäuse des Bohrgeräts vorgesehen ist, und einen Umlenkaufsatz umfasst. Selbstverständlich hat das Bohrgerät wie üblich auch eine Antriebseinheit für die Bohrnadel. Die Bohrnadel, die in dem Gehäuse des Bohrgeräts bzw. Bohrwiderstandsmessgeräts entlang der Bohrerachse zu einer frontseitigen Austrittsöffnung geführt wird, tritt zur Durchführung der Widerstandsmessung dort aus. Erfindungsgemäß ist nun ein Umlenkaufsatz an dieser Frontseite des Gehäuses angeordnet, um die Bohrvorrichtung zu bilden. Bei dieser Anordnung kann sich beim Arbeiten mit dem Gerät die Bohrnadel aus der frontseitigen Austrittsöffnung des Bohrgeräts in und durch den Umlenkaufsatz erstrecken. Der Umlenkaufsatz weist hierzu für die Bohrnadel ein Umlenkgehäuse auf, das in einer Anwendungsanordnung um einen vorbestimmten Winkel gekrümmt ist. Auf diese Weise wird ein aus dem Umlenkaufsatz austretender Abschnitt der Bohrnadel entlang einer Bohrnadelaustrittsachse geführt, die um einen in einer Anwendungsanordnung vorbestimmten Winkel in Bezug auf eine Bohrerachse des Bohrgeräts versetzt ist. So muss das Bohrgerät beispielsweise eben nicht rechtwinklig am Objekt angesetzt werden, um eine rechtwinklig eindringende Bohrung durchzuführen: Der Umlenkaufsatz gestattet hierfür eine Positionierung des Bohrgeräts abweichend von einer rechtwinkligen Anordnung am Objekt, da die Distanz zu einer schwer zugänglichen Stelle durch den Umlenkaufsatz überbrückt wird, sodass die rechtwinklig eindringende Bohrung mittels des Umlenkaufsatzes bequem an der schwer zugänglichen Stelle durchgeführt werden kann.

Unter "Bohrnadel" werden hierin alle Bohrer verstanden, die elastisch biegbar sind, also nach dem Biegen in die Ursprungsform rückgestellt werden. Dabei handelt es sich üblicherweise um Bohrer, deren Schaftlänge um ein Vielfaches größer ist als der Durchmesser und etwa in einem Bereich von 75:1 (Biegeradius Bohrnadel zu Durchmesser Bohrnadel) oder größer liegt, sodass der Bohrer, der üblicherweise aus einem metallischen Werkstoff besteht, elastisch biegbar ist. Die erforderliche Biegbarkeit kann dabei durch geeignete Material- bzw. Legierungswahl für die Bohrnadel unterstützt werden.

Das Bohrgerät ist insofern ein "vorbestimmtes Gerät", weil der Umlenkaufsatz für die Anordenbarkeit bzw. Befestigung an dem Gehäuse eines jeweiligen Bohrgeräts von vornherein entsprechend ausgebildet wird, sodass die jeweiligen Geometrien und auch Befestigungselemente zumindest an dem Umlenkaufsatz derart mit den Geometrien und ggf., falls vorhanden, auch Befestigungselementen am Gehäuse des jeweiligen Bohrgeräts abgestimmt sind, um den Umlenkaufsatz sicher und stabil für die vorgesehenen Arbeiten mit dem Bohrgerät verbinden zu können.

Entsprechend bedeutet "Anwendungsanordnung" auch den Zustand, bei dem Umlenkaufsatz und Bohrgerät arbeitsbereit miteinander gekoppelt sind, und der Umlenkaufsatz die Umlenkung der Bohrnadel um den vorbestimmten Winkel bereitstellt, um eine Bohrung an einer schwer zugänglichen Stelle durchführen zu können.

"Schwer zugängliche Stellen" meint hierin Stellen, an denen eine Bohrung durchgeführt werden soll, die ein Anwender nicht in ergonomischer Weise ohne weiteres mit einem herkömmlichen Bohrgerät erreichen kann, wie beispielsweise bei Bäumen oder Holzmasten im Bereich der Bodengleiche oder über Kopf des Anwenders, auch bspw. bei Balken eines Fachwerks oder aber allgemein bei beliebigen Situationen am Bau oder bei Objekten, die ein Anwender mit einem herkömmlichen Bohrgerät oder Bohrwiderstandsmessgerät nicht ohne Weiteres so erreichen kann, um die Bohrnadel sach- und fachgerecht in das Objekt einführen zu können, insbesondere nicht, wenn die Bohrnadel in einer bestimmten Winkellage in das Objekt eingeführt werden soll. So müssen herkömmliche Bohrgeräte bzw. Bohrwiderstandsmessgeräte zum Einbringen einer rechtwinklig geführten Bohrung auch rechtwinklig zur Objektachse bzw. Objektoberfläche angesetzt werden. "Schwer zugängliche Stellen" befinden sich somit also ober- oder unterhalb eines Arbeitsbereichs, bzw. einer Arbeitsebene, innerhalb dessen/derer ein Anwender eines Bohrgeräts eine unter einer vorbestimmten Winkellage, insbesondere rechtwinklig geführte Bohrung sach- und fachgerecht ohne weitere Hilfsmittel in ergonomischer Haltung durchführen kann. Eine schwer zugängliche Stelle kann zudem auch horizontal außerhalb des Arbeitsbereichs eines Anwenders eines herkömmlichen Bohrgeräts oder Bohrwiderstandsmessgeräts liegen.

Insbesondere kann es sich bei der erfindungsgemäßen Bohrvorrichtung um eine Vorrichtung zur Ausführung von Bohrwiderstandsmessungen handeln, wobei das Bohrgerät ein Bohrwiderstandsmessgerät ist. Bei einem Bohrwiderstandsmessgerät handelt es sich um eine Vorrichtung, die explizit für Bohrwiderstandsmessungen geschaffen, geeignet und ausgebildet ist und die neben der üblichen Antriebseinheit für die Bohrnadel eine Erfassungseinrichtung zur Erfassung des Bohrwiderstands bei der Durchführung einer Bohrung und eine Anzeige- und/oder Speichereinrichtung zur Anzeige und/oder Speicherung des während der Bohrung erfassten Bohrwiderstands aufweist. Auch diese Bohrvorrichtung weist einen Umlenkaufsatz auf, der zum Umlenken der Bohrnadel um den vorbestimmten Winkel an der nadelaustrittsseitigen Frontseite des Gehäuses des Bohrwiderstandsmessgeräts angeordnet ist.

Vorteilhaft kann mit einem Umlenkaufsatz ein Bohrwiderstandsmessgerät, auch ein herkömmliches, nach- oder umgerüstet werden, um Bohrwiderstandsmessungen an schwer zugänglichen Stellen durchzuführen.

Zwar ist denkbar, dass ein Bohrgerät bzw. Bohrwiderstandsmessgerät dauerhaft mit einem Umlenkaufsatz ausgestattet werden kann, der dann unlösbar mit dem Bohrgerät/Bohrwiderstandsmessgerät verbunden wird - es ist jedoch von Vorteil, wenn ein erfindungsgemäßer Umlenkaufsatz lösbar an dem Bohrgerät/Bohrwiderstandsmessgerät angeordnet wird - und entsprechend ausgestaltet ist - sodass der Umlenkaufsatz nach Durchführung der Bohrung an der schwer zugänglichen Stelle auch wieder demontiert werden kann, um das Bohrgerät/Bohrwiderstandsmessgerät wieder für Bohrungen/Bohrwiderstandsmessungen innerhalb des Arbeitsbereichs (also für nicht schwer zugängliche Stellen) eines Anwenders verwenden zu können.

Ferner ist in einer weiteren Ausführungsform vorgesehen, dass bei einer erfindungsgemäßen Bohrvorrichtung der Umlenkaufsatz lösbar mit dem Gehäuse an der Frontseite des Bohrgeräts oder Bohrwiderstandsmessgeräts verbunden ist, sodass der Umlenkaufsatz jederzeit reversibel montiert und demontiert werden kann. Vorteilhaft kann dadurch das Bohrgerät oder Bohrwiderstandsmessgerät auch ohne Umlenkaufsatz betrieben werden, wenn Bohrungen innerhalb des Arbeitsbereichs durchzuführen sind, oder der Umlenkaufsatz kann bei Bedarf, etwa wenn beispielsweise aufgrund der Lage der schwer zugänglichen Stelle ein anderer vorbestimmter Winkel erforderlich ist, ausgetauscht werden, wenn der Umlenkaufsatz ein starres Umlenkgehäuse aufweist. So können sehr viele Typen der aus dem Stand der Technik bekannten Bohrgeräte und Bohrwiderstandsmessgeräte mit einem Umlenkaufsatz zu einer erfindungsgemäßen Bohrvorrichtung nach- und umgerüstet werden.

Damit der Umlenkaufsatz lösbar an der Frontseite des Bohrgeräts oder des Bohrwiderstandsmessgeräts befestigt werden kann, können entsprechende Befestigungsmittel vorgesehen sein, die an dem Ende des Umlenkaufsatzes vorliegen, das dem Bohrgerät oder dem Bohrwiderstandsmessgerät zugewandt ist. Hierbei kann es sich beispielsweise um Klammern oder Rastnasen handeln, die derart ausgebildet sind, dass sie in Eingriff mit Ausnehmungen oder Hinterschnitten kommen können, die bereits am Gehäuse des Bohrgeräts oder des Bohrwiderstandsmessgeräts vorliegen. Alternativ dazu kann auch das Gehäuse des Bohrgeräts oder des Bohrwiderstandsmessgeräts mit Befestigungsmitteln versehen werden, die mit Befestigungsmitteln am Umlenkaufsatz korrespondieren. Beispielsweise kann dazu eine Manschette aus Steck-, Rast-, Klemm-, Schraub- oder Bajonettverschlusselementen an oder nahe der Frontseite des Gehäuses des Bohrgeräts oder des Bohrwiderstandsmessgeräts angebracht werden, die mit entsprechend korrespondierenden Verschlusselementen als Befestigungselemente des Umlenkaufsatzes in Eingriff treten können.

Zur lösbaren Befestigung an einem Bohrgerät oder Bohrwiderstandsmessgerät weist der Umlenkaufsatz an dem Ende, das in der Anwendungsanordnung dem Bohrgerät oder dem Bohrwiderstandsmessgerät zugewandt ist, Befestigungsmittel auf, die entsprechend dem "Gegenteil" ausgebildet sind. Vorteilhaft können die Befestigungsmittel an dem Umlenkaufsatz korrespondierend zu Befestigungsmitteln ausgebildet sein, die an oder nahe der nadelaustrittseitigen Frontseite des Gehäuses des vorbestimmten Bohrgeräts oder des Bohrwiderstandsmessgeräts vorliegen. Besonders vorteilhaft kann der Umlenkaufsatz ein Frontabdeckungselement aufweisen, an dem die Befestigungsmittel vorliegen, die mit den Befestigungsmitteln an der nadelaustrittseitigen Frontseite des Gehäuses des vorbestimmten Bohrgeräts oder des Bohrwiderstandsmessgeräts korrespondieren. "Korrespondieren" heißt dabei, dass die Befestigungsmittel, die an den beiden miteinander zu verbindenden Teilen vorliegen, derart aufeinander abgestimmt sind, dass sie so miteinander in Eingriff zu bringen sind, dass ein zum sachgerechten Ausführen der Arbeiten vorliegender Halt der Teile aneinander gegeben ist.

Das Frontabdeckungselement entspricht in Außenabmessungen und hinsichtlich der dort vorgesehenen Befestigungselemente einer Frontabdeckung eines Bohrgeräts oder Bohrwiderstandsmessgeräts ohne Umlenkaufsatz. Ein solches Frontabdeckungselement weist dann wie eine herkömmliche Frontabdeckung beispielsweise Durchtrittsöffnungen für Schrauben oder Stifte auf, die mit Gewindebohrungen am Gehäuse des Bohrgeräts oder Bohrwiderstandsmessgeräts fluchten, sodass der Umlenkaufsatz nach Abnehmen der Frontabdeckung mittels des Frontabdeckungselements in einfacher Weise an dem Gehäuse befestigt werden kann. So gestaltet sich die Nach- oder Umrüstung eines herkömmlichen Bohrgeräts oder Bohrwiderstandsmessgeräts oder das Austauschen unterschiedlicher Umlenkaufsätze besonders einfach. Vorteilhaft sorgt diese Befestigung für eine exakte Positionierung des Umlenkaufsatzes in Bezug auf den Bohrnadelaustritt am Bohrgerät/Bohrwiderstandsmessgerät.

Eine weitere Ausführungsform der erfindungsgemäßen Bohrvorrichtung sieht vor, dass der Umlenkaufsatz an seinem von den Befestigungsmitteln abgewandten Ende, d. h., an dem Ende, an dem die umgelenkte Bohrnadel austritt, eine Bohrnadelführungsspitze aufweist. Innerhalb dieser Bohrnadelführungsspitze ist vorzugsweise wenigstens ein Zentrierelement vorgesehen, das für eine zentrierte Führung der aus dem Umlenkaufsatz austretenden Bohrnadel in Bezug auf die Bohrnadelaustrittsachse sorgt, um die vorgesehene Bohrung oder Bohrwiderstandsmessung exakt an der vorgesehenen Stelle durchführen zu können.

In einer Ausführungsform kann das Umlenkgehäuse ein starres Umlenkgehäuse sein, das um den vorbestimmten Winkel gekrümmt ist und so immer, auch in der Anwendungsanordnung, die um den vorbestimmten Winkel in Bezug auf die Bohrerachse versetzte Bohrnadelaustrittsachse bereitstellt. Starre Umlenkgehäuse können unterschiedlich gekrümmt sein, sodass Umlenkaufsätze mit unterschiedlichen Winkeln bereitgestellt werden können.

In der Ausführungsform der erfindungsgemäßen Bohrvorrichtung, bei der der Umlenkaufsatz ein starres Umlenkgehäuse aufweist, kann dieses an dem Bohrgerät oder dem Bohrwiderstandsmessgerät zugewandten Ende zudem einen Anschlussführungsadapter für die Bohrnadel aufweisen, der in einer zentrierenden Anschlusshalterung innerhalb des Umlenkgehäuses gehalten wird. Der Anschlussadapter weist eine Durchtrittsöffnung für die Bohrnadel auf. Die Lage der Durchtrittsöffnung korrespondiert mit der Position des Bohrnadelaustritts aus dem Bohrgerät/Bohrwiderstandsmessgerät und fluchtet mit der Bohrerachse, sodass die Bohrnadel in der Anwendungsanordnung aus dem Bohrgerät/Bohrwiderstandsmessgeräts exakt in dem Anschlussführungsadapter des Umlenkgehäuses aufgenommen wird. Der Anschlussführungsadapter kann entsprechend in einer zentrierenden Anschlusshalterung des Umlenkaufsatzes gehalten werden, um die exakte Positionierung der Durchtrittsöffnung im Anschlussadapter an dem Bohrnadelaustritt sicherzustellen, wenn der Umlenkaufsatz in der Anwendungsanordnung an der Frontseite des Bohrgeräts/Bohrwiderstandsmessgeräts angeordnet und befestigt wird.

Zur Führung der in der Anwendung rotierenden Bohrnadel innerhalb des starren Umlenkgehäuses, um die Krümmung der Bohrnadel um den vorbestimmten Winkel so bereitzustellen, dass diese möglichst schwingungsarm geführt wird, kann sich in einer Ausführungsform von der Anschlusshalterung bis zu einer Austrittsöffnung des Umlenkgehäuses, an der die Bohrnadelführungsspitze angeordnet ist, ein gekrümmtes, durchgehendes schlauchartiges Führungselement wie etwa eine flexible Führungshohlwelle, z. B. ein Geflechtschlauch, erstrecken, das zumindest an seinen beiden Enden durch einen Haltering gehalten wird. Das durchgehende Führungselement stellt eine kontinuierliche Führung zur Umlenkung der Bohrnadel bereit und ist hinsichtlich Abmessungen und Material so ausgebildet, dass die rotierende Bohrnadel mit wenig Spiel durch die Krümmung geführt wird. Alternativ zu einem durchgehenden Führungselement können zwischen der Anschlusshalterung und der Austrittsöffnung des Umlenkgehäuses mehrere einzelne Führungs- bzw. Lagerelemente wie beispielsweise Kugellager entlang einem für die Bohrnadel vorgesehenen Krümmungsverlauf in bestimmten Abständen (die von der Anzahl der einzelnen Führungselemente abhängig sind) und in bestimmter Winkellage angeordnet sein, um die Bohrnadel in der vorgesehenen Weise umzulenken, wobei die Bohrnadel zwischen den einzelnen Führungselementen quasi frei läuft. Auch die einzelnen Führungs- bzw. Lagerelemente sind hinsichtlich Abmessungen und Material so ausgebildet, dass die rotierende Bohrnadel mit wenig Spiel durch die Krümmung geführt wird.

Gegebenenfalls kann zur Führung der Bohrnadel im Umlenkaufsatz auch ein durchgehendes Führungselement mit einzelnen Führungselementen kombiniert werden, wobei beispielsweise die einzelnen Führungselemente zur Fixierung der Lage des durchgehenden Führungselements angeordnet werden können.

In einer zur Bohrvorrichtung mit starrem Umlenkgehäuse alternativen Ausführungsform kann das Umlenkgehäuse des Umlenkaufsatzes ein biegsames Umlenkgehäuse sein, das zur Durchführung der Bohrung/Bohrwiderstandsmessung an einer schwer zugänglichen Stelle in die gewünschte Krümmung entsprechend einem vorbestimmten Winkel gebogen werden kann und außerhalb der Anwendung um einen beliebigen Winkel bis zu einem vorgegebenen maximalen Winkel biegbar ist, der von einer Art und einer Länge des biegsamen Umlenkgehäuses abhängig ist. Das biegsame Umlenkgehäuse weist daher nur in der jeweiligen Anwendungsanordnung den vorbestimmen Winkel auf und kann vorteilhaft in unterschiedlichen Winkelpositionen gekrümmt werden, sodass je nach Lage der schwer zugänglichen Stelle immer ein passender Umlenkungswinkel eingestellt werden kann, der eine in einem gewünschten Winkel ausführbare, insbesondere rechtwinklig in das Prüfobjekt eindringende Bohrung gestattet.

Das biegsame Umlenkgehäuse kann beispielsweise durch einen Gelenkschlauch gebildet werden, der vorzugsweise aus kugelgelenkig miteinander verbundenen Schlauchsegmenten bestehen kann. Vorteilhaft kann der Gelenkschlauch nicht nur in einem beliebigen vorbestimmten Winkel eingestellt werden, sondern lässt sich auch in der um den vorbestimmten Winkel eingestellten Biegung durch eine integrierte mechanische Vorhemmung arretieren bzw. fixieren. Diese Fixierung kann jederzeit wieder gelöst werden, sodass der Gelenkschlauch in einem anderen Winkel gekrümmt werden kann. Alternativ zu einem Gelenkschlauch ist eine biegsame Hohlwelle als biegsames Umlenkgehäuse denkbar. Eine biegsame Hohlwelle weist eine oder mehrere Wickellage(n) auf, und kann an ihrem freien Ende - dem in der Anwendungsanordnung von dem Bohrwiderstandsmessgerät abgewandten Ende - eine Halterungshülse aufweisen, an der die biegsame Hohlwelle, die sich nicht in ihrer Winkellage fixieren lässt, händisch oder mittels einer zusätzlichen Halterungsvorrichtung gehalten wird, um den vorbestimmten Winkelversatz einzunehmen, der eine unter einem vorbestimmten Winkel, insbesondere rechtwinklig an der schwer zugänglichen Stelle in das Prüfobjekt eindringende Bohrung ermöglicht.

Gegenüber einer Bohrnadel eines Bohrgeräts oder Bohrwiderstandsmessgeräts ohne Umlenkaufsatz ist die Bohrnadel einer erfindungsgemäßen Bohrvorrichtung bzw. Bohrwiderstandsmessvorrichtung um die Länge des Umlenkaufsatzes verlängert. Weiter ist bei der Wahl der einzusetzenden Bohrnadel zu beachten, dass das verwendete Material eine ausreichende elastische Biegbarkeit aufweist.

Eine erfindungsgemäße Bohrvorrichtung kann damit für Bohrungen an einer vorbestimmten Stelle eines zu überprüfenden Objekts verwendet werden, die außerhalb eines für das Bohrgerät vorgesehenen, wie oben definierten Arbeitsbereichs liegt, indem die Bohrvorrichtung mit dem Umlenkaufsatz derart an der vorgesehenen Stelle angeordnet wird, dass ein aus dem Umlenkaufsatz entlang der Bohrnadelaustrittsachse austretender Abschnitt der Bohrnadel an der vorgesehenen Stelle in einer vorbestimmten Winkellage, insbesondere rechtwinklig in Bezug auf eine Längsachse des zu überprüfenden Objekts eingeführt wird, wobei der Umlenkaufsatz die im Gehäuse des Bohrwiderstandsmessgeräts entlang der Bohrerachse geführte Bohrnadel um einen vorbestimmten Winkel umlenkt.

Ein erfindungsgemäßes Verfahren zur Durchführung von Bohrwiderstandsmessung an schwer zugänglichen Stellen eines Objekts sieht daher die Verwendung einer erfindungsgemäßen Bohrvorrichtung vor und umfasst in einer ersten Ausführungsform die Schritte:
- Anordnen und Befestigen eines Umlenkaufsatzes an der Frontseite des Bohrgeräts oder Bohrwiderstandsmessgeräts, vorzugsweise lösbar Befestigen durch in Eingriff Bringen der Befestigungsmittel des Umlenkaufsatzes mit dem Bohrgerät oder Bohrwiderstandsmessgerät oder mit Befestigungsmitteln des Bohrgeräts oder Bohrwiderstandsmessgeräts;
- Austauschen der Bohrnadel des Bohrgeräts oder Bohrwiderstandsmessgeräts gegen eine um die Länge des Umlenkaufsatzes verlängerte Bohrnadel;
- Ansetzen der Bohrvorrichtung mit dem Umlenkaufsatz an der vorbestimmten Stelle des Objekts, sodass die Bohrnadelaustrittsachse in der vorbestimmten Winkellage, insbesondere rechtwinklig in Bezug auf eine Längsachse oder eine Oberflächenebene angeordnet ist; und
- Durchführen der Bohrung oder Bohrwiderstandsmessung durch eindringen Lassen eines aus dem Umlenkaufsatz entlang der Bohrnadelaustrittsachse austretenden Abschnitts der Bohrnadel an der vorgesehenen Stelle, wobei die Bohrnadel durch den Umlenkaufsatz um den vorbestimmten Winkel in Bezug auf die Bohrerachse umlenkt wird.

Eine weitere Ausführungsform des erfindungsgemäßen Verfahrens sieht vor dem Anordnen und Befestigen des Umlenkaufsatzes das Auswählen und das Bestimmen eines Umlenkaufsatzes vor. Dabei kann das Auswählen beispielsweise aus dem Satz erfolgen, der mit zwei oder mehr Umlenkaufsätzen ausgestattet ist und der in dem erfindungsgemäßen Umrüstset zusammengestellt ist. Das Bestimmen umfasst dabei das Ermitteln eines Winkels, der zum Arbeiten in dem Arbeitsbereich geeignet ist und damit den "vorbestimmten Winkel" bildet, und außerdem umfasst es auch das Ermitteln einer geeigneten Länge des Umlenkaufsatzes und/oder das Ermitteln und Bestimmen geeigneter Befestigungsmittel; stets unter Berücksichtigung eben der Lage der vorbestimmten Stelle (in Bezug auf den Arbeitsbereich), respektive unter Berücksichtigung des Bohrgeräts oder Bohrwiderstandsmessgeräts.

Alternativ oder zusätzlich kann das erfindungsgemäße Verfahren vor dem Anordnen und Befestigen des Umlenkaufsatzes das Entfernen bzw. Lösen eines anderen Umlenkaufsatzes oder einer Frontabdeckung, die an dem vorbestimmten Bohrgerät oder dem vorbestimmten Bohrwiderstandsmessgerät angeordnet sind, umfassen.

Schließlich ist bei Verwendung eines Umlenkaufsatzes mit einem biegsamen Umlenkgehäuse zum Ansetzen der Bohrvorrichtung mit dem Umlenkaufsatz an der vorbestimmten Stelle des Objekts, sodass die Bohrnadelaustrittsachse des Umlenkaufsatzes in der vorbestimmten Winkellage, insbesondere rechtwinklig in Bezug auf eine Längsachse oder eine Oberflächenebene angeordnet ist, der Schritt vorgesehen, dass das biegsame Umlenkgehäuses zum Einstellen des Winkels, der durch die Lage der vorbestimmten Stelle in Bezug auf den Arbeitsbereich vorbestimmt ist, gebogen wird.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung von Ausführungsformen der Erfindung.

Dabei zeigen:
- **Fig. 1**: eine schematische Seitenansicht einer erfindungsgemäßen Bohrvorrichtung mit Bohrgerät und Umlenkaufsatz mit starrem Umlenkgehäuse,
- **Fig. 2**: eine schematische Seitenansicht einer erfindungsgemäßen Bohrvorrichtung mit Bohrgerät und Umlenkaufsatz mit biegsamen Umlenkgehäuse
- **Fig. 3**: eine schematische Seitenansicht einer erfindungsgemäßen Bohrwiderstandsmessvorrichtung bei der Durchführung einer Bohrwiderstandsmessung an einer schwer zugänglichen Stelle im Bereich der Bodengleiche,
- **Fig. 4**: eine perspektivische Ansicht einer erfindungsgemäßen Bohrwiderstandsmess-Vorrichtung bei der Durchführung einer Bohrwiderstandsmessung an einer schwer zugänglichen Stelle im Bereich der Bodengleiche,
- **Fig. 5**: eine schematische Seitenansicht einer erfindungsgemäßen Bohrwiderstandsmessvorrichtung bei der Durchführung einer Bohrwiderstandsmessung an einer schwer zugänglichen Stelle über Kopf,
- **Fig. 6**: eine schematische Seitenansicht eines Umlenkaufsatzes für eine erfindungsgemäße Ausführungsform mit starrem Umlenkgehäuse,
- **Fig. 7**: eine Längsschnittansicht eines Umlenkaufsatzes für eine erfindungsgemäße Ausführungsform mit starrem Umlenkgehäuse,
- **Fig. 8**: eine Detailseitenansicht einer erfindungsgemäßen Bohrwiderstandsmessvorrichtung mit unterschiedlichen Positionen eines Umlenkaufsatzes mit starrem Umlenkgehäuse und vorbestimmtem Winkel,
- **Fig. 9**: eine schematische Seitenansicht einer erfindungsgemäßen Bohrwiderstandsmessvorrichtung mit einem Umlenkaufsatz mit biegsamen Umlenkgehäuse mit einstellbarem und feststellbarem Winkel,
- **Fig. 10**: eine schematische Seitenansicht einer erfindungsgemäßen Bohrwiderstandsmessvorrichtung mit einem Umlenkaufsatz mit biegsamem Umlenkgehäuse auf Basis einer flexiblen Hohlwelle mit einstellbarem Winkel,
- **Fig. 11**: schematische Darstellungen a, b, c von unterschiedlichen Befestigungselementen an Umlenkaufsatz und Gehäuse der Bohrvorrichtung in Schnittansichten und Draufsicht.

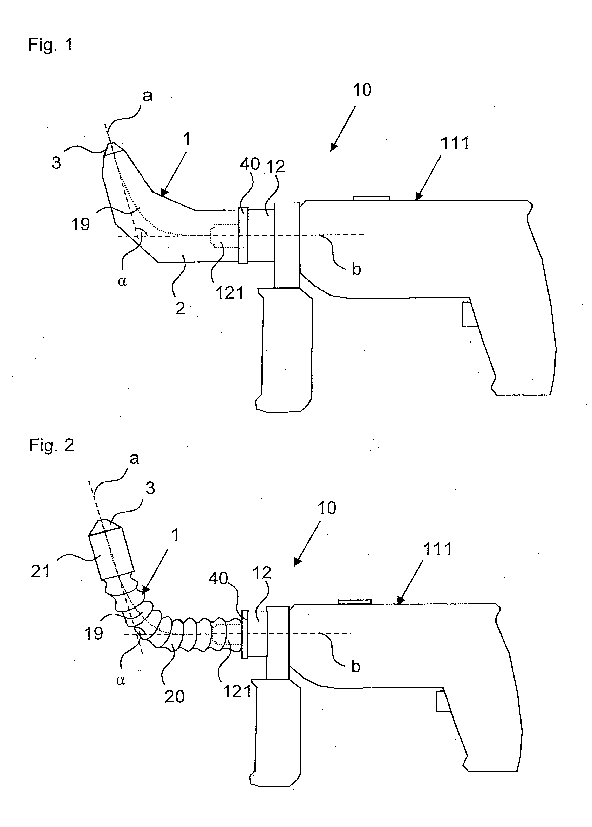

Die erfindungsgemäße Vorrichtung bezieht sich auf eine Bohrvorrichtung 10, die aus einem Bohrgerät 111 oder Bohrwiderstandsmessgerät 11 und einem Umlenkaufsatz 1 besteht, wie er beispielhaft in unterschiedlichen Ausführungsformen in den **Figuren 1 bis 10** dargestellt ist. Mit diesem Umlenkaufsatz 1 kann das herkömmliche Bohrgerät 111 oder das Bohrwiderstandsmessgerät 11 umgerüstet werden, um Bohrungen oder Bohrwiderstandsmessungen auch an schwer zugänglichen Stellen wie im Bereich der Bodengleiche beispielsweise bei Bäumen oder Holzmasten oder über Kopf, etwa bei Balken eines Fachwerks in ergonomischer Arbeitshaltung des Anwenders durchführen zu können. Auch unter der Bodengleiche, etwa bei freigelegten Masten, können mit der erfindungsgemäßen Bohrvorrichtung 10bequem Bohrungen bzw. Widerstandsmessungen durchgeführt werden.

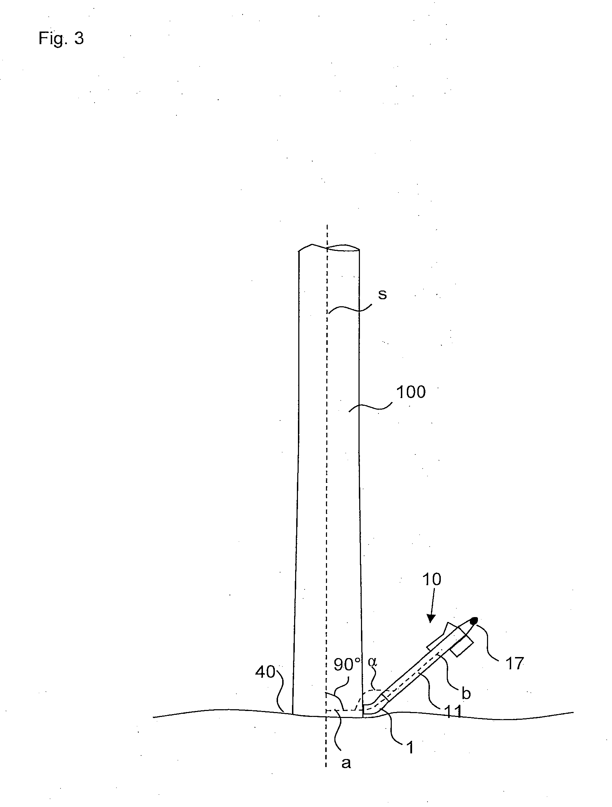

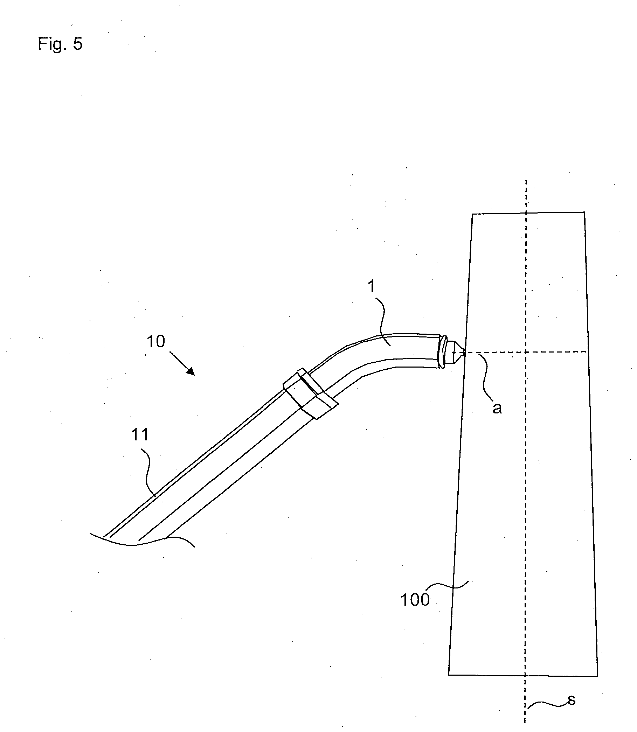

Dabei kann auch problemlos eine zur Mast- bzw. Baum- oder Balkenachse s rechtwinklige oder annähernd rechtwinklige Bohrung durchgeführt werden, wie dies in **Fig. 3** und **5** zu sehen ist. Selbstverständlich können mit der Bohrvorrichtung 10 auch Messbohrungen in einem von einem rechten Winkel abweichenden Winkel durchgeführt werden, falls dies sinnvoll erscheint. So könnte beispielsweise, wenn der Umlenkaufsatz 1 eine Umlenkung der Bohrnadel 19 um einen vorbestimmten, fest eingestellten Winkel bereitstellt, das Bohrwiderstandsmessgerät 11 entsprechend einer Wasserwaage mit einer Libelle zur Ausrichtung in horizontaler oder vertikale Lage ausgestattet sein, sodass eine Messbohrung exakt in dem vorbestimmten, fest eingestellten Winkel erfolgen kann.

In Bezug auf die Bohrwiderstandsmessungen zur Feststellung des inneren Zustands eines Objekts 100 ist aber die im Wesentlichen rechtwinklig angesetzte Messbohrung bevorzugt, da hierbei die Lage möglicher Defekte wie Fäulnis oder ähnliches im Inneren auch ohne komplexe Auswertung einfacher festzustellen ist.

**Fig. 1** und **Fig. 2** zeigen jeweils eine erfindungsgemäße Bohrvorrichtung 10 zur Durchführung von Bohrungen an einer schwer zugänglichen Stelle eines Objekts, das bspw. aus Holz sein kann. Die Bohrvorrichtung 10 umfasst in diesem Beispiel ein Bohrgerät 111, das an seiner Frontseite ein Gehäuseteil 12 aufweist, in dem die gepunktet dargestellte Bohrnadel 19 in einem ebenfalls gepunktet dargestellten Bohrfutter 121, das in dem gezeigten Beispiel aus dem Gehäuseteil 12 herausragt, gelagert, geführt und mit einer nicht dargestellten Antriebseinheit des Bohrgeräts 11 gekoppelt ist. Auf eine detaillierte Darstellung des Bohrgeräts 111 wird hier verzichtet, da im Prinzip jedes handelsübliche Bohrgerät mit einem erfindungsgemäßen Umlenkaufsatz 1 nachgerüstet werden kann, um Bohrungen an unzugänglichen Stellen durchführen zu können, sofern der verwendete Bohrer eine biegsame Bohrnadel 19 ist. Der Umlenkaufsatz 1, der an der Frontseite des Bohrgeräts 111 angeordnet wird, ermöglicht, dass die Bohrnadel 19, die aus dem Gehäuse 12 des Bohrgeräts 111 entlang einer Bohrerachse b geführt wird, um den vorgegebenen Winkel a, der in **Fig. 1** durch das starre Umlenkgehäuse 2 des Umlenkaufsatzes 1 vorgegeben wird und in **Fig. 2** durch das biegsame Umlenkgehäuse 20 eingestellt wird, umgelenkt wird, sodass die Bohrnadel 19 entlang einer Bohrnadelaustrittsachse b aus der Bohrnadelführungsspitze 3 des Umlenkaufsatzes 1 austreten kann, die um den Winkel α zu der Bohrerachse b versetzt ist. Der Umlenkaufsatz 1 ist dabei jeweils mittels eines schematisch dargestellten Befestigungselements 40 an dem Gehäuse 12 des Bohrgeräts 111 befestigt. Unterschiedliche Ausführungsformen möglicher Befestigungselemente werden nachfolgend noch erläutert. Das biegsame Umlenkgehäuse 20 in **Fig. 2** wird durch einen Gelenkschlauch mit einer Austrittshülse 21 gebildet, an die sich die Bohrnadelführungsspitze 3 anschließt.

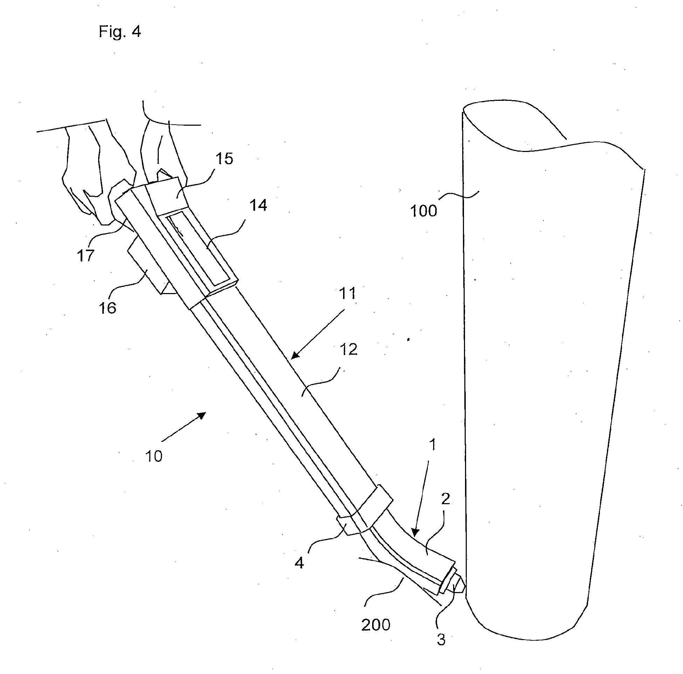

**Fig. 3** zeigt schematisch und **Fig. 4** detaillierter eine erfindungsgemäße Bohrvorrichtung 10 zur Durchführung von Bohrwiderstandsmessungen an einer schwer zugänglichen Stelle eines Baums oder Masts 100, hier im Bereich der Bodengleiche 200 (Erdaufwurf). **Fig. 5** zeigt die Verwendung der Bohrvorrichtung 10 zur Durchführung einer Überkopfmessung.

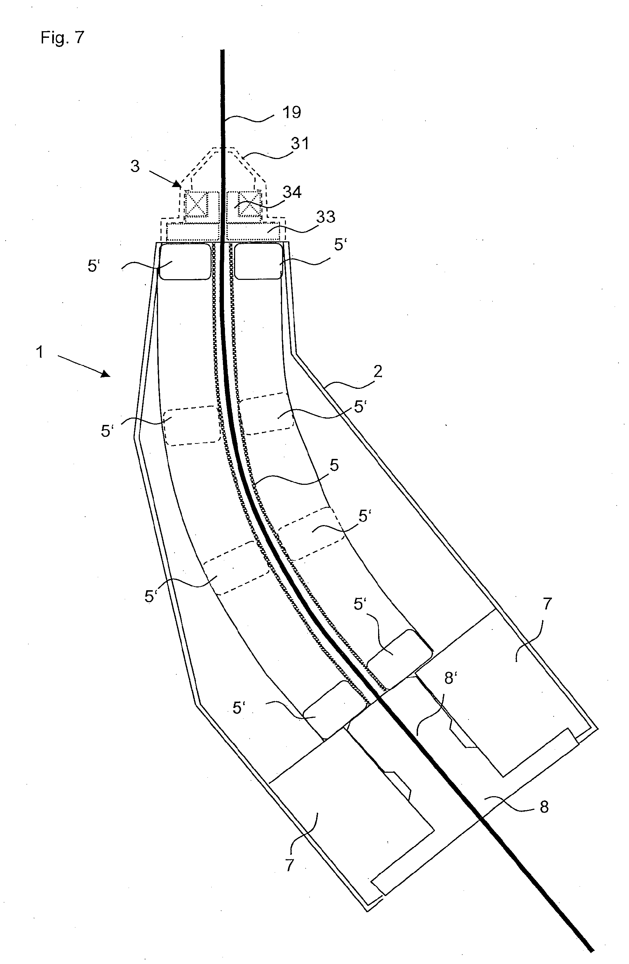

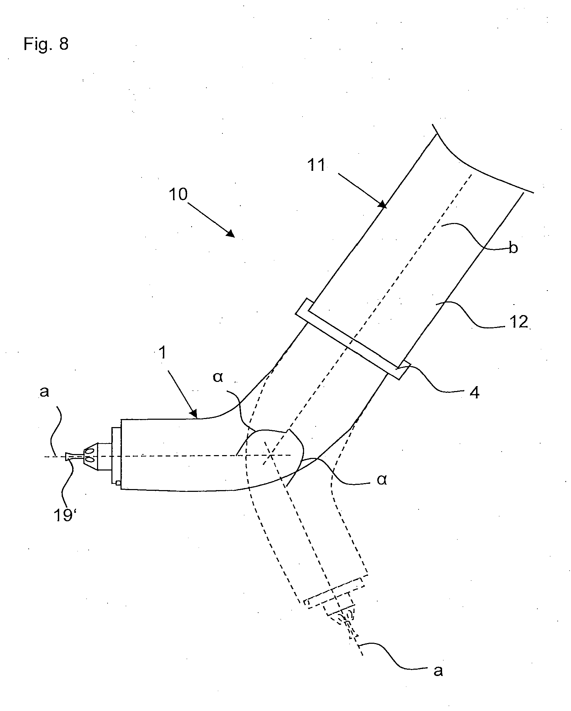

Die Bohrvorrichtung 10 umfasst in diesem Beispiel ein Bohrwiderstandsmessgerät 11, das in herkömmlicher Weise ein Gehäuse 12 und eine Erfassungseinrichtung zur Erfassung des Bohrwiderstands bei der Durchführung einer Bohrwiderstandsmessung aufweist. In dem Gehäuse 12 ist eine Bohrnadel 19 (vgl. **Fig. 7** und **Fig. 8**) gelagert, geführt und mit einer Antriebseinheit 16 gekoppelt. Auf eine detaillierte Darstellung des Bohrwiderstandsmessgeräts 11 wird hier verzichtet, da im Prinzip jedes handelsübliche Bohrwiderstandsmessgerät mit einem erfindungsgemäßen Umlenkaufsatz 1 nachgerüstet werden kann, um Bohrwiderstandsmessungen an unzugänglichen Stellen durchführen zu können. Der Umlenkaufsatz 1, der an der frontseitigen Austrittsöffnung des Bohrwiderstandsmessgeräts 11 angeordnet wird, ermöglicht, dass die Bohrnadel 19, die in dem Gehäuse 12 des Bohrwiderstandsmessgeräts 11 entlang einer Bohrerachse b geführt wird, so umgelenkt wird, dass sie rechtwinklig zur Stamm- bzw. Mastachse s eingeführt werden kann.

In den mit den **Figuren 1** **und** **3 bis 6** gezeigten Beispielen des Umlenkaufsatzes 1 wird die Umlenkung der Bohrnadel 19 um einen vorbestimmten festen Winkel α durch ein starres gekrümmtes Umlenkgehäuse 2 bestimmt. Der Winkel α wird zwischen der Bohrnadelaustrittsachse a, die durch einen aus dem Umlenkaufsatz 1 austretenden Abschnitt der Bohrnadel 19 mit der Bohrnadelspitze 19' definiert wird, und der Bohrerachse b des Bohrgeräts 111 bzw. des Bohrwiderstandsmessgeräts 11 gebildet.

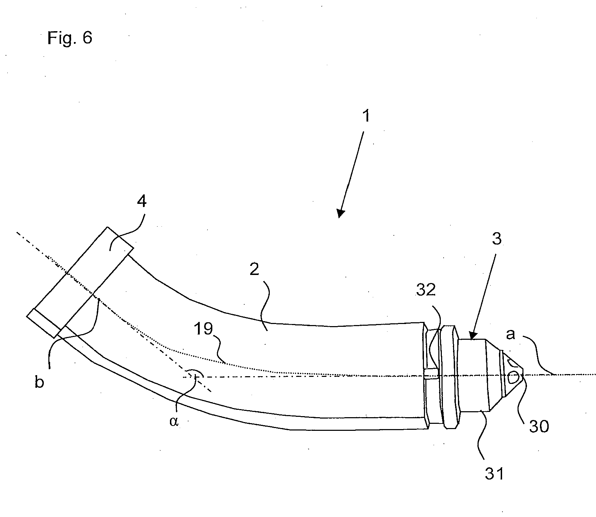

Zur Anordnung des Umlenkaufsatzes 1 an der frontseitigen Austrittsöffnung des Bohrwiderstandsmessgeräts 11 weist der Umlenkaufsatz 1, wie insbesondere in **Fig. 4** **und** **6** dargestellt ist, an dem zu dem Bohrwiderstandsmessgerät 11 weisenden Ende ein Frontabdeckungselement 4 auf. Dieses ist bezüglich der Abmessungen und der Befestigungselemente entsprechend einer Frontabdeckung des Bohrwiderstandsmessgeräts 12 ohne Umlenkaufsatz 1 ausgebildet, sodass zur Umrüstung eines herkömmlichen Bohrwiderstandsmessgeräts 11 einfach dessen Frontabdeckung entfernt und der Umlenkaufsatz 1 mit dem Frontabdeckungselement 4 an dem Gehäuse 12 des Bohrwiderstandsmessgeräts 11 befestigt werden kann. Hierzu können lösbare Steck- oder Rastmechanismen vorgesehen sein, es können aber auch Schraubverbindungen eingesetzt werden. Das in dem vorliegenden Beispiel gezeigte Frontabdeckungselement 4 weist entsprechend dem Gehäuse 12 des Bohrwiderstandmessgeräts 11 einen rechteckigen Querschnitt auf. Selbstverständlich können auch andere Querschnittsformen in Frage kommen, und es ist auch eine Befestigung ohne Frontabdeckungselement 4 denkbar, wobei das Umlenkgehäuse an der Frontseite des Bohrgeräts/Bohrwiderstandsmessgeräts befestigt werden kann, ohne dass dessen Frontabdeckung entfernt wird.

Am anderen Ende des Umlenkaufsatzes 1, an dem die Bohrnadel 19 mit der Bohrnadelspitze 19' austritt, weist, wie in **FIg. 1, 2** **und** **4 bis 8** zu sehen ist, der Umlenkaufsatz 1 eine Bohrnadelführungsspitze 3 auf, deren Gehäuse 31 sich von einem Anschlussflanschabschnitt konusförmig zu einer Bohrnadelaustrittsöffnung 30 hin verjüngt. In diesem Führungsspitzengehäuse 31 sorgen entsprechende Zentrierelemente für einen zentriert geführten Austritt der Bohrnadel 19. In **Fig. 7** sind im Gehäuse 31 der Bohrnadelführungsspitze 3 beispielhaft eine Zentrierscheibe 33 und eine drehbar gelagerte Führungshülse 34 als Zentrierelemente angedeutet. Bei dem weiteren, in **Fig. 6** benachbart zu der Bohrnadelführungsspitze 3 zu sehenden Element 32 handelt es sich um einen Sicherheitsschalter, durch den ein unbeabsichtigtes Austreten der Bohrnadel verhindert wird. Dieser Sicherheitsschalter 32 wird betätigt, um eine erfindungsgemäße Bohrwiderstandsmessvorrichtung 10 einsatzbereit zu machen.

Weiter zeigt **Fig. 7** in Schnittdarstellung eine beispielhafte Ausführungsform eines Umlenkaufsatzes 1, der ein Umlenkgehäuse 2 zur Umlenkung der aus dem hier nicht dargestellten Bohrgerät oder Bohrwiderstandsmessgerät vorgetriebenen Bohrnadel 19 umfasst. In dem Umlenkgehäuse 2 liegt hier an dem dem Bohrgerät oder Bohrwiderstandsmessgerät zugewandten Ende ein Anschlussführungsadapter 8 mit einer Durchtrittsöffnung 8' für die Bohrnadel 19 vor, die die aus dem Bohrgerät bzw. Bohrwiderstandsmessgerät kommende Bohrnadel 19 zentriert geführt aufnimmt. Der Anschlussführungsadapter 8 selbst wird in einer zentrierenden Anschlusshalterung 7 gehalten. Zwischen der Anschlusshalterung 7 und der Austrittsöffnung des Umlenkgehäuses 2, an der die Bohrnadelführungsspitze 3 angeordnet ist, erstreckt sich ein gekrümmtes Führungselement 5 in Form einer Führungshohlwelle, die beidenends jeweils durch einen Haltering gehalten wird. Zugleich ist in derselben **Fig. 7** die alternative Führung der Bohrnadel 19 mittels einzelner Führungselemente 5', beispielsweise Kugellager oder andere Lager, durch Strichelierung angedeutet, wobei - anders als in der Darstellung in **Fig. 7** - auf eine durchgehende Führungshohlwelle 5 verzichtet werden kann. Im gezeigten Beispiel sind die vier einzelnen Führungslager 5' gleichmäßig entlang dem vorgesehenen Umlenkweg für die Bohrnadel 19 verteilt angeordnet, sodass zwei der Führungslager 5' entsprechend den Halteringen in der alternativen Variante mit der durchgehenden Führungswelle 5 an der Austrittsöffnung zur Führungsspitze 3 und an der Anschlusshalterung 7 angeordnet sind, während ein drittes und viertes Führungslager 5' dazwischen um den Krümmungsscheitelpunkt angeordnet sind. In einer Ausführungsform mit drei Führungslagern kann das mittlere Führungslager die Bohrnadel im Krümmungsscheitelpunkt lagern und führen.

Auf die Darstellung von Gehäusestrukturen und Elementen, die zur Anordnung der Führungslager 5' erforderlich sind, wird verzichtet. Lagersitze für die Führungslager 5' können auf übliche Weise ausgebildet sein. Abweichend von dem in **Fig. 7** gezeigten Beispiel können auch mehr als vier Führungslager zur Führung der Umlenkung der Bohrnadel zwischen der Austrittsöffnung und der Anschlusshalterung angeordnet werden. Vorzugsweise werden die Führungslager in gleichmäßigen Abständen voneinander angeordnet, wobei der Abstand von der Anzahl der eingesetzten Führungslager abhängig ist. Dabei hängt die jeweilige Winkellage der Führungslager von der jeweiligen Position entlang dem vorgesehenen Bohrnadelverlauf ab.

Da ferner die Bohrnadel 19 sowohl im Anschlussadapter 8 als auch in der Bohrnadelführungsspitze 3 zentriert geführt werden, kann gegebenenfalls auf die zwei Führungslager 5' verzichtet werden, die entsprechend der Position der Halteringe benachbart zu der Anschlusshalterung 7, die den Anschlussadapter 8 umgibt, und der Führungsspitze 3 angeordnet sind.

Gegebenenfalls können auch beide Varianten, durchgehendes Führungselement 5 und einzelne Führungselemente 5', kombiniert eingesetzt werden.

In **Fig. 8** ist dargestellt, dass ein Umlenkaufsatz 1 in unterschiedlicher Richtung auf einem Bohrwiderstandsmessgerät 11 angeordnet werden kann. Dies ist insofern relevant, als dass bei der Durchführung der Messungen mit der Bohrvorrichtung 10 die Anzeigevorrichtung 14 des Bohrwiderstandsmessgeräts 11 (vgl. **Fig. 4**) gut ablesbar und das Betätigungselement 15 gut erreichbar sein soll. Je nach Lage der schwer zugänglichen Stelle kann so der Umlenkaufsatz 1 in einer dafür günstigeren Position montiert werden. Die Anzahl der möglichen Montagepositionen hängt in erster Linie von der Querschnittsform des Gehäuses 12 des Bohrwiderstandsmessgeräts 11 ab: Im vorliegenden Beispiel hat das Gehäuse 12 einen rechteckigen Querschnitt, sodass sich für den Umlenkaufsatz 1 wie in **FIg. 8** zu sehen ist, zwei mögliche Montagepositionen ergeben.

Nicht dargestellt, aber ohne weiteres übertragbar, sind Ausführungsformen, bei denen das Gehäuse des Bohrwiderstandsmessgeräts beispielsweise einen quadratischen Querschnitt mit folglich vier möglichen Montagepositionen, oder gegebenenfalls auch einen runden Querschnitt aufweist, der - je nach Befestigungsmechanismus - sogar eine beliebige Montageposition eines Umlenkaufsatzes ermöglicht. Wie beispielsweise **FIg. 4** zeigt, kann der feste Winkel des Umlenkgehäuses 2 so gewählt werden, dass die Vorrichtung 10 bequem an einer unzugänglichen Stelle im Bereich der Bodengleiche, auch bei Vorliegen eines Erdaufwurfs 200, durch einen Anwender platziert werden kann, der die Vorrichtung 10 an den Handgriffen 17 des Bohrwiderstandsmessgeräts 11 führt.

Es können als Umrüstset ohne Weiteres eine Vielzahl von Umlenkaufsätzen für ein Bohrwiderstandsmessgerät 11 bereitgestellt werden, deren Umlenkgehäuse Krümmungen mit unterschiedlichen Winkeln α aufweisen, so dass je nach Lage der schwer zugänglichen Stelle ein geeigneter Umlenkaufsatz ausgewählt werden kann. Für eine bequeme Bohrwiderstandsmessung an einem Objekt im Bereich der Bodengleiche ist aufgrund der typischen Länge eines Bohrwiderstandsmessgeräts ein Winkel α von 45° geschickt, wobei der Bediener, ohne sich bücken zu müssen, die Bahrnadelaustrittsöffnung am Stamm oder Mast in Bodenhöhe anordnen kann, um dort eine im Wesentlichen rechtwinklige Messung in Bezug auf die Stamm-/Mastachse durchzuführen. Ein Umrüstset kann aber auch Umlenkaufsätze aufweisen, deren starre Gehäuse von 45° abweichende Winkel von beispielsweise 15°, 30°, 75° oder 90° oder anderen Winkeln aufweisen. Zudem können in einem Umrüstset unterschiedlich lange Umlenkaufsätze enthalten sein, um auch unterschiedliche Distanzen zu den für die Bohrungen vorgesehenen Stellen überbrücken zu können. Schließlich kann ein Umrüstset auch Umlenkaufsätze umfassen, die sich hinsichtlich der Befestigungselemente unterscheiden, so dass verschiedenartige Bohrgeräte und Bohrwiderstandsmessgeräte umgerüstet werden können.

Außer dem Anordnen des Umlenkaufsatzes 1 auf dem Bohrwiderstandsmessgeräts 11 ist zur Umrüstung die Bohrnadel 19 auszutauschen, da für die Bohrvorrichtung 10 mit dem Umlenkaufsatz 1 eine um die Länge des Umlenkaufsatzes 1 verlängerte Bohrnadel 19 erforderlich ist, um die Bohrwiderstandsmessung durchführen zu können. Beim Umrüsten eines Bohrwiderstandsmessgeräts 11 wird daher zunächst die alte Bohrnadel entfernt, und dann kann die Frontabdeckung des Bohrwiderstandsgeräts 11 abmontiert werden. An den entsprechend freigewordenen Befestigungsstellen wird der Umlenkaufsatz mit dem Frontabdeckungselement 4 befestigt, woraufhin die verlängerte Bohrnadel 19 eingesetzt werden kann.

Die Länge der verlängerten zylindrischen Bohrnadel 19 ist somit entsprechend den Abschnitten entlang den Achsen a und b länger als die Länge einer herkömmlichen Bohrnadel, die bereits ein Vielfaches größer ist als deren Durchmesser, der typischerweise im Bereich von 0,5 bis 2,5 mm, bevorzugt von 1,0 mm bis 1,5 mm liegt und auch so für eine verlängerte Bohrnadel 19 eingesetzt wird. Eine bevorzugte Bohrnadelform ist in DE 10 2009 013 069 A1 beschrieben, bei der der zylindrische Nadelschaft über einen Keilabschnitt in eine scharfe und glatte Schneide mündet, damit die Nadel gleichmäßig rotieren kann und verdrängtes Material in Richtung des Nadelschafts verschoben wird, um unerwünschte Kanten, an denen Material hängen bleiben könnte, zu vermeiden. Die Bohrnadelspitze ist nicht rotationssymmetrisch sondern weist in zwei senkrecht aufeinander stehenden Symmetrieebenen unterschiedliche Formen auf: In der ersten Symmetrieebene in Bezug zu einer zentralen Drehachse der Nadel, weist die Nadelspitze eine Schneide mit einer Breite auf, die größer ist als ein Durchmesser des zylindrischen Nadelschafts, wobei der Übergang von dem Nadelschaft entlang des Keilabschnitts zu den beiden Enden der Schneide - die beiden "Flanken" am Nadelkopf - mit einer gleichmäßigen Krümmung verläuft. In der zweiten Symmetrieebene in Bezug zu der zentralen Drehachse, die orthogonal zu der erste Symmetrieebene und durch die zentrale Drehachse verläuft, hat der Keilabschnitt eine geringere Neigung in Bezug auf die zentralen Drehachse als die in einen Grat mündende Schneide. Die Schneide der Bohrnadelspitze 19' weist dabei - in Abhängigkeit des Nadelschaftdurchmessers - vorzugsweise eine Breite in einem Bereich von 0,8 bis 4,0 mm, bevorzugt von 1,0 mm bis 2,0 mm auf.

Alternativ zu der im Zusammenhang mit **Figuren 1** **und** **3 bis 8** beschriebenen Ausführungsform des Umlenkaufsatzes 1 mit einem starren Umlenkgehäuse 2 als Umlenkführungsvorrichtung mit einem festen vorbestimmten Winkel α, sind erfindungsgemäß auch Ausführungen des Umlenkaufsatzes 1 mit biegsamen Hüllvorrichtungen 20,22 als Umlenkgehäuse vorgesehen, die eine Einstellung eines innerhalb vorbestimmter Grenzen beliebigen Winkels α erlauben. Die vorbestimmten Grenzen werden durch einen maximalen Winkel des biegsamen Gehäuses 20,22 bestimmt, der von der Art und Länge des jeweiligen Gehäuses 20,22 abhängig ist. Die Bohrnadel 19 hingegen kann aufgrund ihres geringen Durchmessers im Vergleich zu ihrer Länge beliebig stark gekrümmt werden. Dies kann durch geeignete Materialwahl unterstützt werden.

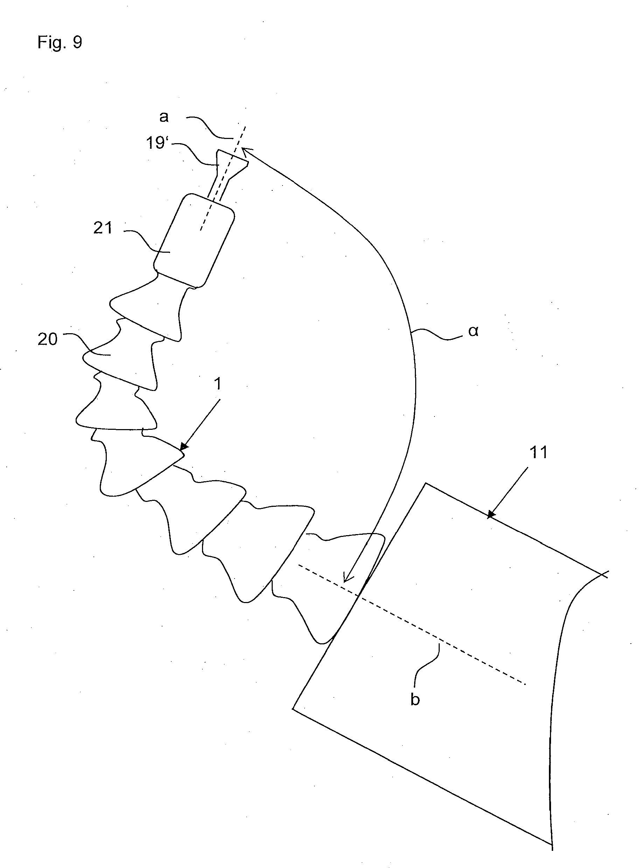

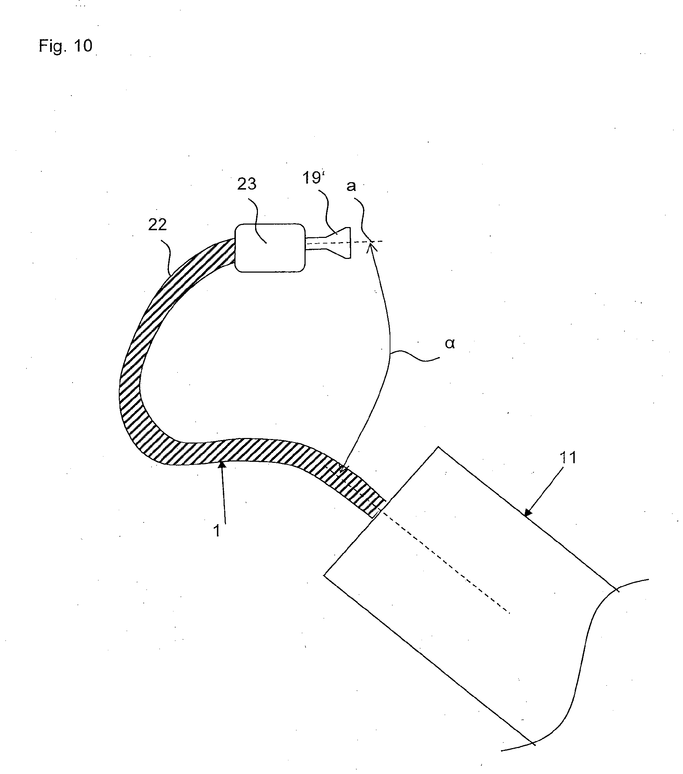

Als Beispiele hierzu sind in **Fig. 2** **und** **9** ein Gelenkschlauch 20 als biegsames Umlenkgehäuse des Umlenkaufsatzes 1 und in **Fig. 10** eine flexible Hohlwelle 22 als biegsames Umlenkgehäuse des Umlenkaufsatzes 1 dargestellt. Der Gelenkschlauch 20 besteht aus kugelgelenkig miteinander verbundenen Schlauchsegmenten, die eine Biegung um einen beliebigen Winkel α zulassen. Der Gelenkschlauch 20 gestattet zudem eine Fixierung in diesem eingestellten Winkel a, die aber auch wieder gelöst werden kann, sodass der Gelenkschlauch 20 in einem anderen Winkel α eingestellt werden kann. Die Bohrnadelaustrittshülse 21 stellt dabei die zentrierte Führung des Abschnitts der Bohrnadel 19 (die sich durch den Gelenkschlauch 20 erstreckt) mit der Bohrnadelspitze 19' bereit. Anders als dargestellt kann alternativ oder zusätzlich hier auch eine Führungsspitze 3 wie in den obig beschriebenen Ausführungen mit dem Umlenkgehäuse 2 vorgesehen sein.

In ähnlicher Weise erstreckt sich die Bohrnadel 19 durch die biegsame Hohlwelle 22, die aus einer oder mehreren Wickellagen aufgebaut ist. Gegebenenfalls kann die äußerste Wickellage von einem elastischen Schutzschlauch umgeben sein. Da sich die biegsame Hohlwelle 22 nicht in einem eingestellten Winkel α feststellen lässt, ist hier an dem freien Ende der biegsame Hohlwelle 22 eine Hülse 23 vorgesehen, die nicht nur als zentrierende Führungsvorrichtung für die austretende Bohrnadel 19 dient, sondern auch als Haltevorrichtung dient. Der Bohrnadelaustritt kann damit händisch geführt und der Winkel α händisch eingestellt werden.

Zwar ist dies in **Fig. 9** **und** **10** nicht dargestellt, aber auch hier kann der Umlenkaufsatz 1 zur Verbindung mit dem Gehäuse 12 des Bohrwiderstandsmessgeräts 11 ein Frontabdeckungselement 4 aufweisen, das bei der Umrüstung anstelle der Frontabdeckung an der frontseitigen Austrittsöffnung des Bohrwiderstandsmessgeräts 11 angeordnet wird. Alternativ dazu kann der Umlenkaufsatz allerdings auch Befestigungselemente ohne ein spezielles Frontabdeckungselement aufweisen, um den Umlenkaufsatz ohne Entfernung von Gehäuseteilen des Bohrgeräts an dessen Frontseite zu befestigen.

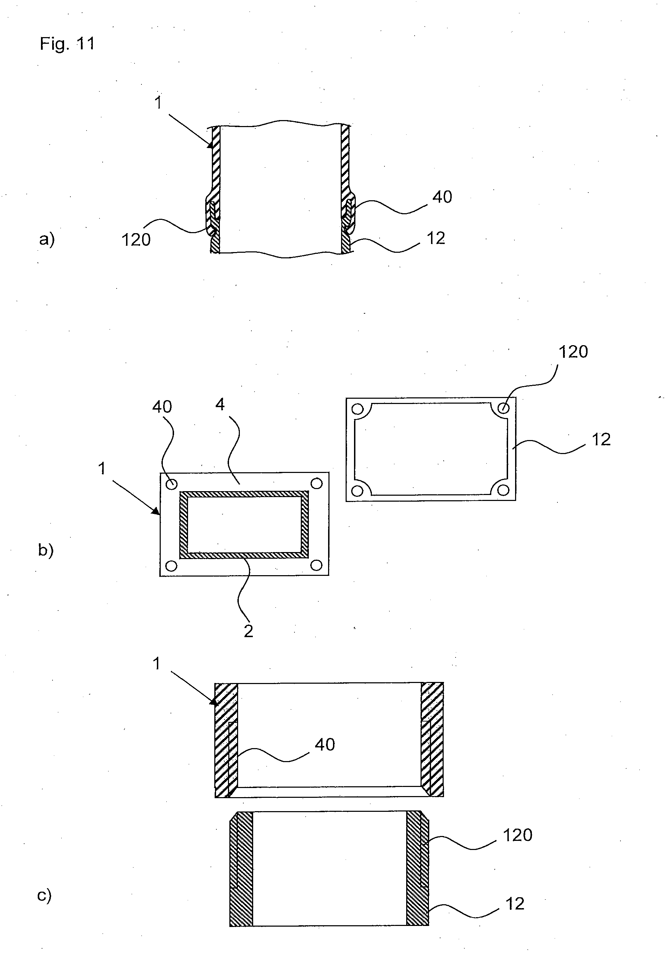

In **Fig. 11** sind drei Beispiele a, b und c für verschiedene Befestigungsvarianten eines Umlenkaufsatzes 1 an einem Bohrgerät oder Bohrwiderstandsmessgerät dargestellt.

In a) ist als Befestigungsvariante ein Steck- bzw. Rastverschluss skizziert, bei dem ein zu dem hier nicht dargestellten Bohrgerät oder Bohrwiderstandsmessgerät weisender Endabschnitt des Umlenkaufsatzes 1 und das Gehäuse 12 des Bohrgeräts als Befestigungselemente korrespondierend ausgeformte Rastelemente 40, 120 aufweisen. In b) ist in Draufsicht der Umlenkaufsatz 1 und versetzt dazu die Frontseite des Gehäuses 12 mit rechteckigem Querschnitt eines Bohrgeräts oder Bohrwiderstandsmessgeräts zu sehen, bei dem eine Frontabdeckung entfernt wurde. Der Umlenkaufsatz 1 in b) weist ein Frontabdeckungselement 4 auf, wobei das Umlenkgehäuse 2, das sich aus der Blattebene erstrecken würde, aus Übersichtsgründen geschnitten dargestellt ist. Das Frontabdeckungselement 4 entspricht in seiner Form und den Abmessungen der entfernten Frontabdeckung und weist Durchtrittsöffnungen 40 als Befestigungselemente auf, die bei Anlage des Umlenkaufsatzes 1 an dem Gehäuse 12 mit den dort vorhandenen Aufnahmebohrungen 120 als korrespondierende Befestigungselemente fluchten, so dass in diesem Beispiel der Umlenkaufsatz 1 mittels Schrauben oder Stiften befestigt werden kann, die durch die dann fluchtenden Durchtrittsöffnungen 40 und Aufnahmebohrungen 120 eingebracht werden. In c) ist eine beispielhafte Variante gezeigt, in der der Umlenkaufsatz 1 an dem dem Gehäuse 12 zugewandten Ende ein Gewinde 40 als Befestigungselement aufweist, das mit einem korrespondierenden Gegengewinde 120 am Gehäuse 12 in Eingriff gebracht werden kann.

Die in **Fig. 11** gezeigten Beispiele sollen allerdings in keiner Weise beschränkend verstanden werden - vielmehr sollen als Befestigungselemente alle denkbaren Klemm-Steck-, Rast-, Schraub- oder Bajonett-Verschlüsse etc. umfasst sein, mit denen ein erfindungsgemäßer Umlenkaufsatz - mit oder ohne Frontabdeckungselement - an einem Bohrgerät oder Bohrwiderstandsmessgerät, bzw. dessen Gehäuse - mit oder ohne Frontabdeckung - lösbar befestigt werden kann. Gegebenenfalls kann die Wahl der geeigneten Befestigungselemente durch eine Form des Gehäuses und der ggf. korrespondierenden Form des Umlenkgehäuses bzw. des Frontabdeckungselement eingeschränkt sein. So ist ein Bajonett- oder Schraubverschluss nur bei kreisrunden Querschnitten realisierbar. Gegebenenfalls kann das Gehäuse eines Bohrgeräts oder Bohrwiderstandsmessgeräts auch mit Befestigungselementen wie mit Aufnahmen oder Rastausnehmungen und anderen geeigneten Elementen ergänzt werden, um mit einem Umlenkaufsatz nachgerüstet werden zu können.

Es ist in erster Linie zwar vorgesehen, dass ein Umlenkaufsatz zur Bildung einer erfindungsgemäßen Bohrvorrichtung zur Bohrung oder Bohrwiderstandsmessung an schwer zugänglichen Stellen an der frontseitigen Austrittsöffnung des Bohrgeräts bzw. Bohrwiderstandsmessgeräts zum Nach- bzw. Umrüsten reversibel verbunden, gelöst und ausgetauscht werden kann, um somit eine breiteste Anwendungspalette mit einem Bohrwiderstandsmessgerät abdecken zu können, indem beispielsweise auch unterschiedlich gewinkelte Aufsätze und das Bohrwiderstandmessgerät ohne Umlenkaufsatz eingesetzt werden können. Es ist aber auch denkbar, dass eine erfindungsgemäße Bohrvorrichtung zur Durchführung einer Bohrung oder Bohrwiderstandsmessung an einer schwer zugänglichen Stelle als ein Bohrgerät oder Bohrwiderstandsmessgerät mit einem nicht lös- oder austauschbaren Umlenkaufsatz ausgebildet ist. In einem solchen Fall bietet sich die Variante mit einem biegsamen Umlenkgehäuse aus einem Gelenkschlauch oder einer biegsamen Hohlwelle oder entsprechenden Vorrichtungen an.

Ferner kann eine erfindungsgemäße Vorrichtung mit Elementen ausgestattet sein, die das Tragen, Aufstellen oder Platzieren unterstützen und erleichtern, insbesondere bei Überkopfmessungen oder bei den Ausführungsformen mit biegsamer Umlenkführungsvorrichtung, vor allem der biegsamen Hohlwelle, die sich nicht in einem vorbestimmten Winkel feststellen lässt, sondern eine zusätzliche Führung der Austrittshülse erfordert, die entweder händische erfolgt oder zusätzliche Halterung nötig macht. Außer den in **Fig. 3** **und** **4** dargestellten Handgriffen 17 kann eine erfindungsgemäße Vorrichtung beispielsweise mit einer Stativvorrichtung nahe der schwer zugänglichen Stelle platziert werden, sodass die biegsame Umlenkführungsvorrichtung bequem per Hand eingestellt oder geführt werden kann. Umgekehrt könnte die biegsame Umlenkführungsvorrichtung an der Austrittshülse beispielsweise mit einer Manschette oder einer Klammer am Objekt angesetzt werden, sodass das Bohrwiderstandsmessgerät der erfindungsgemäßen Vorrichtung gehalten werden kann. Andere Ansätze können Tragegurte oder Auflagepolster vorsehen, um das Bohrwiderstandsmessgerät der erfindungsgemäßen Vorrichtung rucksackähnlich auf dem Rücken oder über einer Schulter an der Seite tragen oder auf eine Schulter aufschultern zu können, und so die Hände frei zu haben, um die biegsame Umlenkführungsvorrichtung einzustellen und/oder zu führen. Es wird bemerkt, dass die anhand der Beispiele mit einer Bohrwiderstandsmessvorrichtung gezeigten Ausführungen auch auf eine Bohrvorrichtung übertragbar sind, die nicht zur Ausführung von Widerstandsmessungen sondern lediglich zum Einbringen von Bohrungen ausgebildet sind, und umgekehrt.

### BEZUGSZEICHENLISTE

- 1: Umlenkaufsatz
- 2: starres Umlenkgehäuse
- 20: Gelenkschlauch als biegsames Umlenkgehäuse
- 21: Austrittshülse
- 22: biegsame Welle als biegsames Umlenkgehäuse
- 23: Halterungshülse
- 3, 30, 31: Führungsspitze, Bohrnadelaustrittsöffnung, Gehäuse
- 32, 33, 34: Sicherheitsschalter, Zentrierscheibe, Zentrierhülse
- 4: Frontabdeckungselement
- 40: Befestigungsmittel (Durchtrittsöffnungen, Rastelemente), Umlenkaufsatz
- 5, 5': durchgehendes, einzelnes Führungselement
- 7: Anschlusshalterung
- 8, 8': Anschlussführungsadapter, Durchtrittsöffnung
- 10: Bohrvorrichtung/Bohrwiderstandsmessvorrichtung
- 11,111: Bohrwiderstandsmessgerät, Bohrgerät
- 12: Gehäuse eines Bohrwiderstandsmessgeräts/Bohrgeräts
- 120: Befestigungsmittel Gehäuse Bohrwiderstandsmessgerät/Bohrgerät
- 121: Bohrfutter
- 14: Anzeigevorrichtung
- 15: Betätigungselement
- 16: Antriebseinheit
- 17: Handgriff
- 19, 19': Bohrnadel, Bohrnadelspitze
- 200: Bodengleiche, Erdaufwurf
- 100: Mast/Baum
- a: Bohrnadelaustrittsachse des Umlenkaufsatzes
- b: Bohrerachse des Bohrgeräts/Bohrwiderstandsmessgeräts
- s: Achse Mast/Baum
- α: Winkel zwischen a und b

## Patentansprüche

1. Bohrvorrichtung (10), aufweisend
- ein Bohrgerät (111) oder ein Bohrwiderstandsmessgerät (11) und
- eine in dem Bohrgerät (111) oder dem Bohrwiderstandsmessgerät (11) gelagerte und entlang einer Bohrerachse (b) geführte Bohrnadel (19), die aus einem Gehäuse (12) des Bohrgeräts (111) oder Bohrwiderstandsmessgeräts (11) frontseitig austritt,
**dadurch gekennzeichnet, dass**
die Bohrvorrichtung (10)
- einen Umlenkaufsatz (1) aufweist,
der an einer Frontseite des Gehäuses (12) des Bohrgeräts (111) oder Bohrwiderstandsmessgeräts (11) angeordnet ist und der ein Umlenkgehäuse (2,20,22) aufweist, in dem die Bohrnadel (19) geführt ist, wobei das Umlenkgehäuse (2,20,22) für die Bohrnadel (19) in einer Anwendungsanordnung um einen vorbestimmten Winkel (α) gekrümmt ist, wobei ein aus dem Umlenkaufsatz (1) austretender Abschnitt der Bohrnadel (19) entlang einer Bohrnadelaustrittsachse (a) führbar ist, die um den in der Anwendungsanordnung vorbestimmten Winkel (α) in Bezug auf die Bohrerachse (b) des Bohrgeräts (111) oder Bohrwiderstandsmessgeräts (11) versetzt ist.

2. Bohrvorrichtung (10) nach Anspruch 1, wobei
der Umlenkaufsatz (1) lösbar mit dem Gehäuse (12) an der Frontseite des Bohrgeräts (111) oder des Bohrwiderstandsmessgeräts (11) mittels Befestigungsmitteln (40) verbunden ist, die an dem Ende des Umlenkaufsatzes (1) vorliegen, das dem Bohrgerät (111) oder dem Bohrwiderstandsmessgerät (11) zugewandt ist, wobei die Befestigungsmittel (40) bevorzugt mit Befestigungsmitteln (120) korrespondieren, die an oder nahe der Frontseite des Gehäuses (12) des Bohrgeräts (111) oder des Bohrwiderstandsmessgeräts (11) vorliegen,
wobei die Befestigungsmittel (40) des Umlenkaufsatzes (1) besonders bevorzugt an einem Frontabdeckungselement (4) vorliegen, dessen Abmessungen korrespondierend zu den Abmessungen des Gehäuses (12) an der Frontseite des Bohrgeräts (111) oder Bohrwiderstandsmessgeräts (11) ausgebildet sind.

3. Bohrvorrichtung (10) nach Anspruch 1 oder 2,
**dadurch gekennzeichnet, dass**
der Umlenkaufsatz (1) an seinem von den Befestigungsmitteln (40) abgewandten Ende eine Bohrnadelführungsspitze (3) aufweist, die bevorzugt zumindest ein Zentrierelement (33,34) aufweist.

4. Bohrvorrichtung (10) nach zumindest einem der Ansprüche 1 bis 3,
**dadurch gekennzeichnet, dass**
das Umlenkgehäuse
- ein starres Umlenkgehäuse (2) mit einer Krümmung um einen vorbestimmten Winkel (α) ist.

5. Bohrvorrichtung (10) nach Anspruch 4,
**dadurch gekennzeichnet, dass**
in dem starren Umlenkgehäuse (2) an dem dem Bohrgerät (111) oder Bohrwiderstandsmessgerät (11) zugewandten Ende ein Anschlussführungsadapter (8) für die Bohrnadel (19) vorgesehen ist, die in einer zentrierenden Anschlusshalterung (7) des Umlenkaufsatzes (1) gehalten wird, wobei
- sich von der Anschlusshalterung (7) bis zu einer Austrittsöffnung des Umlenkgehäuses (2), an der die Bohrnadelführungsspitze (3) angeordnet ist, ein gekrümmtes Führungselement (5) erstreckt, das zumindest an seinen beiden Enden durch einen Haltering gehalten wird, und/oder
- zwischen der Anschlusshalterung (7) und der Austrittsöffnung des Umlenkgehäuses (2) eine Mehrzahl einzelner Führungselemente (5') entlang einem für die Bohrnadel (19) vorgesehenen Krümmungsverlauf angeordnet ist.

6. Bohrvorrichtung (10) nach zumindest einem der Ansprüche 1 bis 3,
**dadurch gekennzeichnet, dass**
das Umlenkgehäuse ein biegsames Umlenkgehäuse ist, das um einen Winkel (α) bis zu einem vorgegebenen maximalen Winkel biegbar ist,
wobei bevorzugt das biegsame Umlenkgehäuse
- ein Gelenkschlauch (20) aus miteinander verbundenen Schlauchsegmenten ist, bevorzugt aus kugelgelenkigen miteinander verbundenen Schlauchsegmenten, wobei besonders bevorzugt der Gelenkschlauch (20) in einer um den vorbestimmten Winkel (α) eingestellten Biegung arretierbar ist, oder
- eine biegsame Hohlwelle (22) aus zumindest einer Wickellage ist, wobei bevorzugt die biegsame Hohlwelle (22) an ihrem freien Ende eine Halterungshülse (23) aufweist.

7. Verfahren zur Durchführung von Bohrungen oder Bohrwiderstandsmessungen an einer vorbestimmten Stelle eines Objekts (100) unter Verwendung einer Bohrvorrichtung (10) nach zumindest einem der Ansprüche 1 bis 6,
**umfassend die Schritte**
- Anordnen und Befestigen des Umlenkaufsatzes (1) an der Frontseite des Bohrgeräts (111) oder Bohrwiderstandsmessgeräts (11) mittels an dem Umlenkaufsatz (1) und dem Bohrgerät (111) oder dem Bohrwiderstandsmessgerät (11) vorliegenden Befestigungsmitteln (40,120),
- Austauschen der Bohrnadel des Bohrgeräts (111) oder des Bohrwiderstandsmessgeräts (11) gegen eine um die Länge des Umlenkaufsatzes (1) verlängerte Bohrnadel (19),
- Ansetzen der Bohrvorrichtung (10) mit dem Umlenkaufsatz (1) an der vorbestimmten Stelle des Objekts (100), sodass die Bohrnadelaustrittsachse (a) des Umlenkaufsatzes (1) in einer vorbestimmten Winkellage, vorzugsweise rechtwinklig in Bezug auf eine Längsachse (s) oder eine Oberflächenebene der Stelle angeordnet ist, und
- Durchführen der Bohrung oder Bohrwiderstandsmessung durch eindringen Lassen eines aus dem Umlenkaufsatz (1) entlang der Bohrnadelaustrittsachse (a) austretenden Abschnitts der Bohrnadel (19) an der vorgesehenen Stelle, wobei die Bohrnadel (19) durch den Umlenkaufsatz (1) um den vorbestimmten Winkel (α) in Bezug auf die Bohrerachse (b) umgelenkt wird.

8. Verfahren nach Anspruch 7,
**umfassend die Schritte**
vor dem Anordnen und Befestigen des Umlenkaufsatzes (1) Ermitteln eines Winkels (α) und Vorbestimmen einer Länge eines geeigneten Umlenkaufsatzes unter Berücksichtigung der Lage einer Stelle zur Durchführung der Bohrung in einem Arbeitsbereich und dann
- Auswählen eines Umlenkaufsatzes (1), bevorzugt aus einem Satz von zwei oder mehr Umlenkaufsätzen (1) eines Umrüstsets nach Anspruch 10, mit dem Winkel (α) und/oder einer vorbestimmten Länge und/oder vorbestimmten Befestigungsmitteln (40), und/oder
- Entfernen eines anderen Umlenkaufsatzes (1) oder einer Frontabdeckung, die an dem vorbestimmten Bohrgerät (111) oder dem vorbestimmten Bohrwiderstandsmessgerät (11) angeordnet sind.

9. Verfahren nach Anspruch 7 oder 8, wobei bei Verwendung eines Umlenkaufsatzes mit einem biegsamen Umlenkgehäuse (20,22) zum Ansetzen der Bohrvorrichtung (10) mit dem Umlenkaufsatz (1) an der vorbestimmten Stelle des Objekts (100), das biegsame Umlenkgehäuse (20,22) zum Einstellen des Winkels (α) gebogen wird.

## Claims

1. Drilling apparatus (10) comprising
- a drilling device (111) or a drilling resistance measuring device (11) and
- a drilling needle (19) that is supported and guided along a drill axis (b) in the drilling device (111) or the drilling resistance measuring device (11) and exits from the housing (12) of the drilling device (111) or drilling resistance measuring device (11) at the front side,
**characterized in that**
the drilling apparatus (10)
- comprises a deflector attachment (1) which is arranged at the front side of the housing (12) of the drilling device (111) or drilling resistance measuring device (11),
and comprises a deflector housing (2, 20, 22) in which the drilling needle (19) is guided,
wherein the deflector housing (2, 20, 22) for the drilling needle (19) in the arrangement of use is curved about a predetermined angle (α) wherein a section of the drilling needle (19) exiting from the deflector attachment (1) can be guided along the drilling needle exit axis (a) that is offset in the arrangement of use by the predetermined angle (α) in relation to the drill axis (b) of the drilling device (111) or drilling resistance measuring device (11).

2. Drilling apparatus (10) according to claim 1, wherein
the deflector attachment (1) is connected detachably to the housing (12) at the front side of the drilling device (111) or of the drilling resistance measuring device (11) by means of fastening means (40) which are present at the end of the deflector attachment (1) that is facing the drilling device (111) or the drilling resistance measuring device (11), wherein the fastening means (40) correspond preferably with fastening means (120) that are present at or near the front side of the housing (12) of the drilling device (111) or of the drilling resistance measuring device (11),
wherein the fastening means (40) of the deflector attachment (1) are present particularly preferred at a front cover element (4) whose dimensions are embodied corresponding to the dimensions of the housing (12) at the front side of the drilling device (111) or drilling resistance measuring device (11).

3. Drilling apparatus (10) according to claim 1 or 2,
**characterized in that**
the deflector attachment (1) at its end that is facing away from the fastening means (40) comprises a drilling needle guiding tip (3) that comprises preferably at least one centering element (33, 34).

4. Drilling apparatus (10) according to at least one of claims 1 to 3,
**characterized in that**
the deflector housing
- is a rigid deflector housing (2) with a curvature about a predetermined angle (α).

5. Drilling apparatus (10) according to claim 4,
**characterized in that**
in the rigid deflector housing (2) at the end that is facing the predetermined drilling device (111) or drilling resistance measuring device (11) a connecting guiding adapter (8) for the drilling needle (19) is provided which is held in a centering connecting holder (7) of the deflector attachment (1), wherein
- a curved guiding element (5) that is held at least at its two ends by a holder ring extends from the connecting holder (7) to an exit opening of the deflector housing (2) at which the drilling needle guiding tip (3) is arranged, and/or
- between the connecting holder (7) and the exit opening of the deflector housing (2) a plurality of individual guiding elements (5') are arranged along a curvature course provided for the drilling needle (19).

6. Drilling apparatus (10) accordingat least one of to claims 1 to 3,
**characterized in that**
the deflector housing is a bendable deflector housing that is bendable about an angle (α) up to a predetermined maximum angle,
wherein preferably the bendable deflector housing
- is an articulated hose (20) of hose segments that are connected to each other, preferably of hose segments connected to each other by ball joints wherein particularly preferred the articulated hose (20) can be arrested in a bent position adjusted to the predetermined angle (α), or
- a bendable hollow shaft (22) of at least one wound layer, wherein preferably the bendable hollow shaft (22) comprises a holding sleeve (23) at its free end.

7. Method for carrying out drilling or drilling resistance measurements at a predetermined location of an object (100) by use of a drilling apparatus (10) according to claim 1 to 6,
**comprising the steps**
- arranging and fastening a deflector attachment (1) at the front side of the predetermined drilling device (111) or drilling resistance measuring device (11) by means of fastening means (40, 120) present at the deflector attachment (1) and the predetermined drilling device (111) or the drilling resistance measuring device (11),
- exchanging the drilling needle of the drilling device (111) or of the drilling resistance measuring device (11) for a drilling needle (19) that is extended by the length of the deflector attachment (1),
- placing the drilling apparatus (10) with the deflector attachment (1) against the predetermined location of the object (100) so that the drilling needle exit axis (a) of the deflector attachment (1) is arranged at a predetermined angular position, preferably at a right angle in relation to a longitudinal axis (s) or a surface plane at the location, and
- carrying out drilling or drilling resistance measurement by allowing a section of the drilling needle (19) exiting from the deflector attachment (1) along the drilling needle exit axis (a) to penetrate at the predetermined location, wherein the drilling needle (19) is deflected by the deflector attachment (1) about the predetermined angle (α) in relation to the drill axis (b).

8. Method according to claim 7,
**comprising the steps**
prior to arranging and fastening the deflector attachment (1) determining an angle (α) and predetermining a length of a suitable deflector attachment by taking into consideration the position of a location for carrying out drilling in a working range and then
- selecting a deflector attachment (1), preferably from a set of two or more deflector attachments (1) of a conversion set according to claim 10, with the angle (α) and/or a predetermined length and/or predetermined fastening means (40), and/or
- removing a different deflector attachment (1) or a front cover that are arranged at the predetermined drilling device (111) or the predetermined drilling resistance measuring device (11).

9. Method according to claim 7 or 8, wherein, when using a deflector attachment with a bendable deflector housing (20, 22) for placing the drilling apparatus (10) with the deflector attachment (1) against the predetermined location of the object (100), the bendable deflector housing (20, 22) is bent for adjusting the angle (α).

## Revendications

1. Dispositif de perçage (10), présentant
- une perceuse (111) ou un instrument de mesure de la résistance de perçage (11) et
- une aiguille de perçage (19) montée dans la perceuse (111) ou dans l'instrument de mesure de la résistance de perçage (11), guidée le long d'un axe du foret (b) et sortant côté frontal d'un carter (12) de la perceuse (111) ou de l'instrument de mesure de la résistance de perçage (11),
**caractérisé en ce que**
le dispositif de perçage (10)
- présente un embout de renvoi (1)
qui est disposé sur un côté frontal du carter (12) de la perceuse (111) ou de l'instrument de mesure de la résistance de perçage (11),
et qui présente un carter de renvoi (2, 20, 22) dans lequel l'aiguille de perçage (19) est guidée, le carter de renvoi (2, 20, 22) étant recourbé d'un angle (α) prédéfini pour l'aiguille de perçage (19) dans une disposition d'application, une section de l'aiguille de perçage (19) sortant de l'embout de renvoi (1) pouvant être guidée le long d'un axe de sortie de l'aiguille de perçage (a), qui est décalé de l'angle (α) prédéfini dans la disposition d'application par rapport à l'axe du foret (b) de la perceuse (111) ou de l'instrument de mesure de la résistance de perçage (11).

2. Dispositif de perçage (10) selon la revendication 1,
l'embout de renvoi (1) étant relié de manière amovible au carter (12) sur le côté frontal de la perceuse (111) ou de l'instrument de mesure de la résistance de perçage (11) à l'aide de moyens de fixation (40) qui se trouvent à l'extrémité de l'embout de renvoi (1) qui est tourné vers la perceuse (111) ou l'instrument de mesure de la résistance de perçage (11), les moyens de fixation (40) correspondant préférablement à des moyens de fixation (120) qui se trouvent sur ou près du côté frontal du carter (12) de la perceuse (111) ou l'instrument de mesure de la résistance de perçage (11),
les moyens de fixation (40) de l'embout de renvoi (1) se trouvant plus préférablement sur un élément du panneau frontal (4), dont les dimensions sont conçues pour correspondre aux dimensions du carter (12) sur le côté frontal de la perceuse (111) ou de l'instrument de mesure de la résistance de perçage (11).

3. Dispositif de perçage (10) selon la revendication 1 ou 2,
**caractérisé en ce que**
l'embout de renvoi (1) présente, à son extrémité située à l'opposé des moyens de fixation (40), une pointe de guidage de l'aiguille de perçage (3) qui présente préférablement au moins un élément de centrage (33, 34).

4. Dispositif de perçage (10) selon au moins l'une des revendications 1 à 3,
**caractérisé en ce que**
le carter de renvoi
- est un carter de renvoi rigide (2) avec une courbure d'un angle (α) prédéfini.

5. Dispositif de perçage (10) selon la revendication 4,
**caractérisé en ce que**
un adaptateur de guidage de raccordement (8) est prévu à l'extrémité tournée vers la perceuse (111) ou l'instrument de mesure de la résistance de perçage (11) dans le carter de renvoi rigide (2), pour l'aiguille de guidage (19) qui est maintenue dans un support de raccordement (7) centrant de l'embout de renvoi (1),
- un élément de guidage (5) incurvé et maintenu au moins à ses deux extrémités par un anneau de retenue, s'étendant du support de raccordement (7) jusqu'à un orifice de sortie du carter de renvoi (2), sur lequel est disposée la pointe de guidage de l'aiguille de perçage (3), et/ou
- une majorité d'éléments de guidage (5') étant disposée le long d'un profil de courbure prévu pour l'aiguille de perçage (19) entre le support de raccordement (7) et l'orifice de sortie du carter de renvoi (2).

6. Dispositif de perçage (10) selon au moins l'une des revendications 1 à 3,
**caractérisé en ce que**
le carter de renvoi est un carter de renvoi flexible, pouvant être plié d'un angle (α) jusqu'à un angle maximal prédéfini,
le carter de renvoi flexible étant préférablement
- un flexible articulé (20), composé de segments de flexible reliés, préférablement de segments de flexible reliés par une articulation à rotule, le flexible articulé (20) pouvant être plus préférablement bloqué dans une flexion définie par l'angle (α) prédéfini ou
- un arbre creux flexible (22), composé au moins d'une couche enroulée, l'arbre creux flexible (22) présentant préférablement, à son extrémité libre, une douille de support (23).

7. Procédé de réalisation de trous ou de mesures de la résistance de perçage à un endroit prédéfini d'un objet (100) au moyen d'un dispositif de perçage (10) selon au moins l'une des revendications 1 à 6,
**comprenant les étapes**
- disposition et fixation de l'embout de renvoi (1) sur le côté frontal de la perceuse (111) ou de l'instrument de mesure de la résistance de perçage (11) à l'aide de moyens de fixation (40, 120) présents sur l'embout de renvoi (1) et la perceuse (111) ou l'instrument de mesure de la résistance de perçage (11),
- remplacement de l'aiguille de perçage de la perceuse (111) ou de l'instrument de mesure de la résistance de perçage (11) par une aiguille de perçage (19) prolongée de la longueur de l'embout de renvoi (1),
- application du dispositif de perçage (10) avec l'embout de renvoi (1) à l'endroit prédéfini de l'objet (100) de sorte que l'axe de sortie de l'aiguille de perçage (a) de l'embout de renvoi (1) soit disposé dans une position angulaire prédéfinie, préférablement perpendiculaire à un axe longitudinal (s) ou à un plan de surface de l'endroit et
- réalisation du perçage ou de la mesure de la résistance de perçage par pénétration d'une section de l'aiguille de perçage (19), sortant de l'embout de renvoi (1) le long de l'axe de sortie de l'aiguille de perçage (a), à l'endroit prévu, l'aiguille de perçage (19) étant déviée de l'angle (α) prédéfini par rapport à l'axe du foret (b) par l'embout de renvoi (1).

8. Procédé selon la revendication 7,
**comprenant les étapes**
avant la disposition et la fixation de l'embout de renvoi (1), détermination d'un angle (α) et prédéfinition d'une longueur d'un embout de renvoi approprié compte tenu de la position d'un endroit pour la réalisation du trou dans une zone de travail, puis
- sélection d'un embout de renvoi (1), préférablement à partir d'un ensemble de deux ou plusieurs embouts de renvoi (1) d'un kit de rééquipement selon la revendication 10, avec l'angle (α) et/ou une longueur prédéfinie et/ou des moyens de fixation prédéfinis (40) et/ou
- retrait d'un autre embout de renvoi (1) ou d'un panneau frontal, disposés sur la perceuse (111) prédéfinie ou sur l'instrument de mesure de la résistance de perçage (11) prédéfini.

9. Procédé selon la revendication 7 ou 8, le carter de renvoi flexible (20, 22) étant courbé pour la définition de l'angle (α) au moyen d'un embout de renvoi avec un carter de renvoi flexible (20, 22) pour l'application du dispositif de perçage (10) avec l'embout de renvoi (1) à l'endroit prédéfini de l'objet (100).
